(19) **Europäisches Patentamt / European Patent Office / Office européen des brevets**

(11) **EP 3 740 120 B1**

(12) **EUROPEAN PATENT SPECIFICATION**

(45) Date of publication and mention
of the grant of the patent:
**15.07.2026 Bulletin 2026/29**

(21) Application number: **19706323.3**

(22) Date of filing: **18.01.2019**

(51) International Patent Classification (IPC):
*A61B 5/00* *(2006.01)* *A61B 5/0215* *(2006.01)*

(52) Cooperative Patent Classification (CPC):
**A61B 5/02152; A61B 5/686;** A61B 2562/0247;
A61B 2562/028

(86) International application number:
**PCT/US2019/014108**

(87) International publication number:
**WO 2019/143876 (25.07.2019 Gazette 2019/30)**

(54) **MEMS DEVICE FOR AN IMPLANT ASSEMBLY**

MEMS-VORRICHTUNG FÜR EINE IMPLANTATANORDNUNG

DISPOSITIF MEMS POUR ENSEMBLE IMPLANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.01.2018 US 201862618848 P**

(43) Date of publication of application:
**25.11.2020 Bulletin 2020/48**

(73) Proprietor: **Endotronix, Inc.**
**Lisle, IL 60532 (US)**

(72) Inventors:
• **ZAMAN, Mohammad Faisal**
**Naperville, Illinois 60565 (US)**
• **FONG, Jeffrey**
**Chicago, Illinois 60608 (US)**
• **CHEE, Julian**
**De Pere, Wisconsin 54115 (US)**
• **PANIAN, Tyler**
**Naperville, Illinois 60563 (US)**
• **NAGY, Michael**
**Lombard, Illinois 60148 (US)**

(74) Representative: **Müller-Boré & Partner**
**Patentanwälte PartG mbB**
**Friedenheimer Brücke 21**
**80639 München (DE)**

(56) References cited:
**US-A1- 2012 197 155 US-A1- 2016 324 443**

EP 3 740 120 B1

**Description**

CROSS-REFERENCE TO RELATED APPLICATIONS

This application claims priority to and the benefit of each the following applications: U.S. Provisional Patent Application No. 62/618,848 entitled "MEMS DEVICE FOR AN IMPLANT ASSEMBLY," filed on January 18, 2018. This application is also a continuation-in-part of U.S. Patent Application No. 15/213,712 entitled "PRESSURE SENSING IMPLANT" filed on July 19, 2016 which is a continuation-in-part of U.S. Patent Application No. 14/777,654 entitled "PRESSURE SENSING IMPLANT" filed on September 16, 2015 which is a national phase entry application that claims priority to International Patent Application No. PCT/US2014/030661 filed March 17, 2014 which claims priority to Provisional Patent Application No. 61/786,793 entitled "PRESSURE SENSING IMPLANT," filed on March 15, 2013. U.S. Patent Application No. 15/837,075 entitled "IMPLANTABLE SENSOR ENCLOSURE WITH THIN SIDEWALLS," filed on December 11, 2017, which claims priority to U.S. Patent Application No. 14/129,725 (U.S. Patent No. 9,867,552) entitled "IMPLANTABLE SENSOR ENCLOSURE WITH THIN SIDEWALLS," filed on February 21, 2014, which claims priority to International Patent Application No. PCT/US/2012/044998 entitled "IMPLANTABLE SENSOR ENCLOSURE WITH THIN SIDEWALLS," filed on June 29, 2012 which claims priority to Provisional Patent Application No. 61/502,982 entitled "IMPLANTABLE SENSOR ENCLOSURE WITH THIN SIDEWALLS," filed on June 30, 2011.

FIELD OF INVENTION

This application relates to implanted sensor packages and more particularly to an improved structure for implanted sensor packages.

BACKGROUND

Implantable wireless sensors are useful in assisting diagnosis and treatment of many diseases. Examples of wireless sensor readers are disclosed in U.S. Patent No. 8,154,389, and US Patent No. 8,493,187, each entitled Wireless Sensor Reader. In particular, there are many applications where measuring pressure from within a blood vessel deep in a patient's body is clinically beneficial. For example, measuring the pressure in the heart's pulmonary artery is helpful in optimizing treatment of heart failure and pulmonary hypertension. In this type of application, a sensor may need to be implanted up to 20 cm beneath the surface of the skin.

One type of implant for this application is described by US Patent Application 15/213,712 ("the '712 application", published as US 2016/0324443 A1) which illustrates the use of floating bases and vented cavities within a hermetically sealed implant housing. Notably, these types of implants include micro-electromechanical systems (MEMS) having capacitive electrodes that transduce pressure to capacitance. The implants include a capacitive sensor that includes a flexible diaphragm and a less flexible (more rigid) base, with a cavity in between and capacitive electrodes at least partially extending within the cavity. The sensor may be fabricated having a diaphragm formed on one wafer and a base formed on a second wafer, in which the base is attached to the diaphragm around a perimeter. Two layers - diaphragm and base - thus form a capacitive sensor in which the base has a smaller overall area than the diaphragm. The sensor may then be attached to an implant housing by inserting the smaller-area base into a larger housing cavity of the implant housing and is hermetically sealed therein leaving an outer surface of the diaphragm exposed to the environment outside of the implant housing. The attachment line between the base and diaphragm within the implant housing may be referred to as a "bond line" and the cavity defined between the diaphragm and base may be identified as a "capacitive gap." The '712 application discusses various embodiments of these devices.

US 2012/197155 A1 describes an implantable capacitive pressure sensor apparatus and method for making such an apparatus including a first pressure sensor portion and a second pressure sensor portion. The first pressure sensor portion includes a diaphragm electrode connectable to ground (e.g., the diaphragm electrode being positioned in close proximity to the body when implanted therein such that the diaphragm electrode is deformable in response to pressure applied thereto by the body). The second pressure sensor portion includes a signal electrode (e.g., wherein the first pressure sensor portion and the second pressure sensor portion are coupled such that a gap is provided between the diaphragm electrode and the signal electrode) and an insulator material. The signal electrode is provided on and in direct contact with the insulator material to electrically isolate the signal electrode such that parasitic capacitance effects on the signal electrode are reduced.

Known processes for bonding the two wafers together, forming the capacitive gap, generally cause various internal stresses at the bond line (i.e. point of bonding about the perimeter between the base and diaphragm). As such, a high-stress line may be formed around the perimeter of the cavity. The stress may relax over time slowly, and/or change with temperature. The stress may case the implant to sense or to generate inaccurate readings in capacitance over the life of the device as capacitance is changing in response to a parameter (e.g. stress or temperature) other than the desired measurement (e.g. pressure). Stress relaxation along the bond line may be a slow change over time which can cause signal drift due to slow change in capacitive gap height.

It has been identified that the physical features of the capacitive gap may be important to the functional performance of the pressure sensing implant. The capacitance, and hence the resonant frequency of the implant, depends on the change in distance between the capacitive electrodes. Thus, it is desirable to have an implant whose capacitance changes when the distance between the capacitive electrode (the gap height) changes, due to an external pressure. However, it is not desirable to also experience capacitance changes due to any other reason, including: slow changes in the composition of gas (or fluid) in the capacitive gap over time; slow changes in distance between plates due to relaxation of built-in internal stresses over time; and changes in temperature. These composition changes may cause error in implant readings because capacitance (and hence resonant frequency) may be changing for reasons other than due to external pressure. In one embodiment, the design of the implant is subject to a heightened level of sensitivity as a gap change of 1 percent (i.e. 10 nanometers) over time may be significant enough to put the implant out of its designed range or specified functionality.

Additionally, for implants with vented cavities as discussed above, there is a need for the composition of the gap fluid (i.e. liquid or gas) to stabilize quickly once the diaphragm and base wafer are hermetically sealed to the cavity of the implant housing. If, for example, a component within the implant housing (such as a PCB) outgasses water vapor into the housing cavity, and the vent between the housing cavity and capacitive gap is only a small hole, it may take a long time for the capacitive gap to achieve homogeneity with the outer housing cavity, causing long term inaccuracy while the stabilization (homogeneity) takes place.

Further challenges have been identified related to the practical implementation of such an implant. Particularly, it has been identified that an implant designed to meet the criteria above may also be sensitive to temperature and pressure changes, including while in ambient conditions, when positioned within a patient, and during transition between ambient conditions to in vivo. These temperature and pressure variations may cause the sensor to communicate inconsistent signals or otherwise function in an inconsistent manner. As such, in addition to providing an implant that includes desirable features including having high sensitivity, good electrical isolation between electrical nodes and surrounding body fluids or tissue, being highly stable over time, having good mechanical strength, incorporating biocompatible materials, minimizing the use of ferritic materials; the implant may also include a design that would increase mechanical strength and reduce the effects of changes to temperature and pressure stresses.

Thus, it is desirable to provide an improved implant to optimize desirable features and to ensure a consistent and reliable operation over the life of the implant. Further, because MEMS fabrication processes can be complex and require very long development times, it is desirable to identify designs that allow modifying performance specifications with only minor changes in structure. Such specifications for an implant include: sensitivity, full scale range, frequency offset, and avoidance of pull-in between capacitive electrodes. As such, there is a need for a capacitive MEMS pressure sensor with floating base and vented cavity that provides: reduced gap height error due to relaxation of internal stresses at the bond line; reduced gap height error due to changes in stress at the bond line, due to temperature changes; reduced capacitance error due to slow, long-term change of composition in the fluid (gas or liquid) in the gap; and to provide a cost-effective means to simply modify and control key design parameters such as sensitivity, full-scale range, maximum voltage withstand (pull-in resistance), and temperature sensitivity.

Thus, according to an aspect, the objective relates to an implant and the method of assembling the implant. This objective is achieved by the subject-matter of the independent claim 1. Particular embodiments are defined in the dependent claims.

SUMMARY OF THE INVENTION

This application relates to hermetically packaged wireless electronics and more particularly to an implantable sensor design and manufacturing approach to optimize manufacturability, size, longevity, RF characteristics, and overall performance while minimizing potential effects of operating in various pressure conditions, temperature conditions and manufacturing induced stress conditions. The invention is defined in the appended claims.

In an embodiment, an implant comprises a diaphragm which may be configured to flex in response to external changes in pressure and a base, which may be a floating base attached to said diaphragm to define a capacitive gap between said diaphragm and said floating base. Said attachment may be positioned along a perimeter of said capacitive gap wherein said floating base is positioned entirely within said cavity and wherein said attachment along said perimeter contains at least one discontinuity. The floating base may be attached only to said diaphragm. The sensor may be a capacitive pressure sensor. The diaphragm may be connected to said housing to form a hermetic seal about said housing cavity. The sensor may include at least one electrical contact positioned on said diaphragm. The at least one discontinuity may vent said capacitive gap to said housing cavity such that liquid or gas may pass between said gap and said housing cavity.

The at least one discontinuity may allow at least one electrical trace to connect at least one said electrical contact outside of said capacitive gap to at least one electrode positioned at least partially within said capacitive gap. The capacitive gap may include electrodes positioned on said diaphragm and said floating base. The implant may include a coil in electrical communication with said sensor, said coil positioned within said housing. The diaphragm may be made of a glass material and the floating base may be made of silicon. The diaphragm may include a thickness of about 195μm and said at least one discontinuity may include a length of about 6.75 mm. The location and size of said at least one discontinuity may be configured to enhance at least one performance parameter of said implant. The at least one performance parameter may be selected from the following list: sensitivity to pressure, sensitivity to temperature, mechanical strength, and long term accuracy. Sensitivity to temperature may be improved by positioning said at least one discontinuity such that a coefficient of thermal expansion mismatch effect counteracts a gas expansion effect to reduce capacitive gap change due to temperature.

In another embodiment, provided is a method of assembling an implant The method comprises providing a housing that defines a cavity. A floating base may be attached to a diaphragm to form a capacitive gap, said attachment being positioned along a perimeter of said capacitive gap and including at least one discontinuity to enhance at least one performance parameter of said implant. The diaphragm may be attached to said housing such that said floating base is positioned within said cavity. A coil may be attached to said sensor. A bottom may be attached to said housing to form a hermetic seal about said cavity. The diaphragm may be hermetically attached to said housing by at least one laser weld about the perimeter of said cavity. The diaphragm may be made of a glass material and said floating base may be made of silicon.

In another embodiment, provided is an implant that comprises a housing that defines a cavity and having a sensor connected to said housing. The sensor comprising a diaphragm may have at least one diaphragm electrode. A base may be attached to said diaphragm, said base including at least one base electrode wherein said base and diaphragm define a capacitive gap between the at least one diaphragm electrode and the at least one base electrode. The base may attach to said diaphragm along a perimeter of said capacitive gap, said attachment including at least one discontinuity configured to enhance at least one performance parameter of said implant. The base may be positioned within the cavity of said housing. The base may further include at least one through hole to provide access to electrically connect said at least one electrode to a component outside said capacitive gap. The diaphragm may include a thickness between about 100μm to about 300μm. The implant may include a circuit having a resonant frequency that changes in response to a sensed parameter such as pressure. The implant may be filled with a liquid or gel or may be a vacuum or filed with a predetermined pressure. The base may include at least one through substrate via (TSV) to electrically connect the at least one of the base electrode and said diaphragm electrode to a component outside said capacitive gap. The diaphragm may include a thick region and a thin region wherein said thin region is aligned with said capacitive gap. The at least one performance parameter may be selected from the following list: sensitivity to pressure, sensitivity to temperature, mechanical strength, and long term accuracy. The sensitivity to temperature may be achieved by positioning said at least one discontinuity such that a coefficient of thermal expansion mismatch effect counteracts a gas expansion effect to reduce capacitive gap change due to temperature.

In yet another embodiment, provided is an implant that comprises a housing, which may have a plurality of walls and at least one opening. A sensor may be connected to an opening in said housing. Said sensor may comprise a first layer having a first dimension and a second layer having a second dimension shorter than said first dimension. Said second layer may be positioned entirely within said housing and a surface of said first layer is exposed to the exterior of said housing. Said second layer may be attached to said first layer to define a capacitive gap between layers. Said attachment may be positioned along a perimeter of said capacitive gap. Said attachment along said perimeter may include at least one discontinuity. The location and position of said at least one discontinuity may be configured to enhance at least one performance parameter of said implant.

BRIEF DESCRIPTION OF THE DRAWINGS

Embodiments of the present disclosure are described herein with reference to the drawings wherein:

Figure 1 is a perspective view of an embodiment for a wireless implant of the present disclosure;

Figure 2 is a schematic cross sectional view of a sensor component of the wireless implant of the present disclosure;

Figure 3 is a schematic cross-sectional view of the implant of Figure 1;

Figure 4 is a bottom schematic view of a portion of a partially assembled implant with a printed circuit board for an embodiment of the implant;

Figure 5A is a top schematic view of a known sensor component of the implant separate from an implant housing;

Figure 5B is a top perspective schematic view of the known sensor component of the instant separate from the implant housing;

Figure 6 is a top schematic view of a sensor component of the instant disclosure separate from the implant housing;

Figure 7 is a top perspective schematic view of the sensor component of the instant disclosure separate from the implant housing;

Figure 8 is a graph that represents frequency v. pressure derived from simulations of known capacitor sensor;

Figure 9 includes a top graph that represents capacitance v. discontinuity length and a bottom graph that represents frequency v. discontinuity length of the implant of the instant disclosure;

Figure 10 is a graph that represents frequency v. pressure derived from simulations of an embodiment of the implant of the instant application;

Figure 11 is a graph that represents change in diaphragm displacement due to temperature v. axis length derived from simulations of embodiments of the implant of the instant application;

Figure 12 is a graph that represents change in diaphragm displacement due to temperature v. axis length derived from simulations of embodiments of the implant of the instant application;

Figure 13 is a graph that represents change in diaphragm displacement due to temperature v. axis length derived from simulations of embodiments of the implant of the instant application;

Figure 14 is a graph that represents change in diaphragm displacement due to temperature v. axis length derived from simulations of embodiments of the implant of the instant application;

Figure 15 is a graph that represents change in diaphragm displacement due to temperature v. axis length derived from simulations of embodiments of the implant of the instant application; and

Figure 16 is a flowchart illustrating a method of assembling an implant in accordance with the present disclosure.

DETAILED DESCRIPTION OF THE PREFERRED EMBODIMENTS

Reference will now be made in detail to embodiments of the invention, examples of which are illustrated in the accompanying drawings. It is to be understood that other embodiments may be utilized and structural and functional changes may be made without departing from the respective scope of the invention.

This application relates to an implant 10 and more particularly to an implantable sensor design and manufacturing approach to optimize manufacturability, size, longevity, RF characteristics, Q, and overall performance related to increasing sensitivity and minimizing thermally induced frequency offset. To improve the device design and to minimize thermally induced frequency offset, the implant may be constructed to optimize deflection of the surfaces of the implant capacitor while providing ample protection from the environment and maximizing space for the internal components such as the coil.

In one embodiment, the instant application refers to an improvement to the interaction of a bond line 74 and a capacitive gap 64 that exist between a diaphragm wafer 60 and a floating base wafer 80 described above and in application 15/213,712. The term "floating base" used in the instant application is illustrated in the '712 application and may refer to a wafer or layer that is attached or structurally supported by a wall (e.g. the diaphragm) of an implant housing and includes a smaller dimension than the wall. The floating base may appear to float within the housing cavity as it is structurally supported by just the diaphragm. In particular, it was identified that the performance of various pressure sensing implant designs may be manipulated by adjusting structural relationships between the continuity of the bond line 74 and the size of the capacitive gap 64 that exist between the diaphragm and base. The discovered relationship may assist with designing pressure sensing implants within various design constraints that improve the long term performance of the implant.

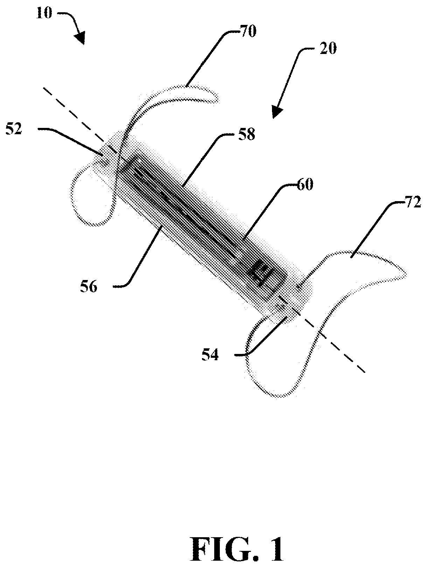

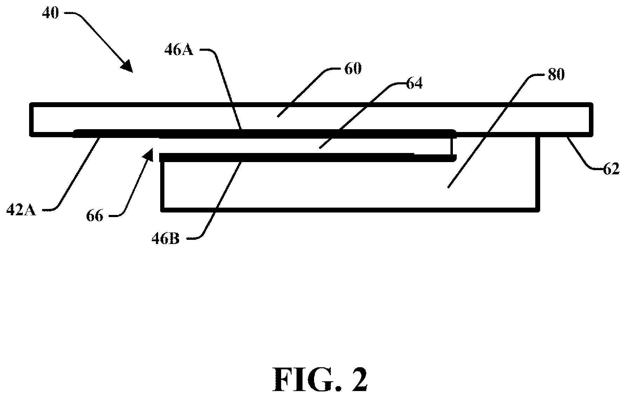

Figures 1 through 5 illustrate a wireless implant housing 20 that is described by the '712 application. Here, the sensor 40 may include the top wall or diaphragm 60 having electronic components placed thereon. The top wall 60 may remain a diaphragm once bonded together with the remaining side walls of the sensor housing 20. The sensor 40 may be a MEMS type sensor. The sensor 40 may be a capacitive type sensor, formed by attaching a base 80 to the diaphragm 60. In one embodiment, the capacitive gap 64 may be positioned between the base 80 and the diaphragm 60. At least one of the base 80 and the diaphragm 60 may be etched to create the capacitive gap 64 at least partially between the base 80 and the diaphragm 60. Electrodes 46A, 46B may be patterned on either side of the capacitive gap 64 (See Figures 2, and 4-6). Electrode 46A may be placed on the diaphragm 60 and electrode 46B may be placed on the base 80. Electrode 46A may terminate to bond pads 42A, 42B which may be available to connect the electrode 46A to other components in the implant 10. Note that the embodiments depicted in Figures 1-5, there are two electrodes 46A, 46B positioned on base 80. Together, they form two capacitors in series. Other electrode combinations and configurations are possible, including those disclosed in the '712 application.

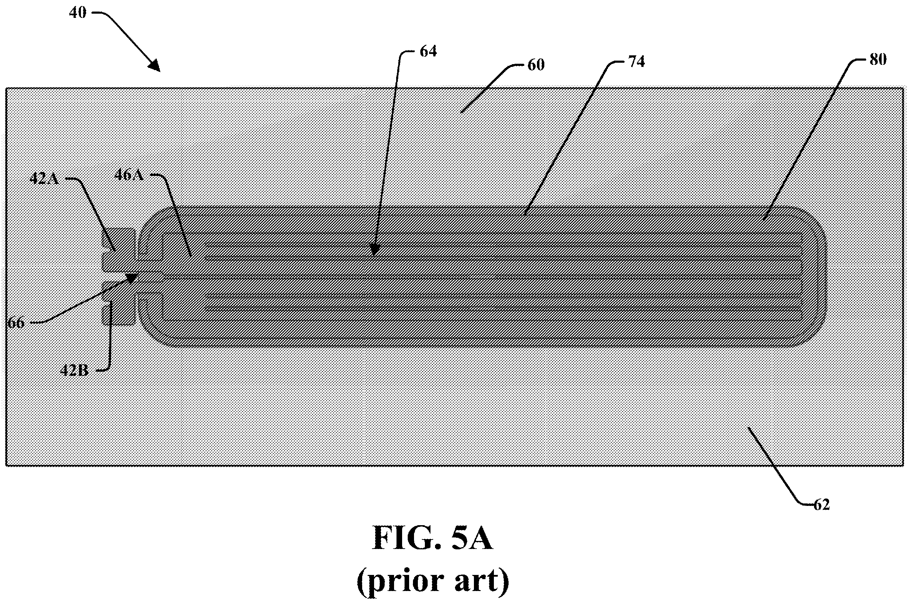

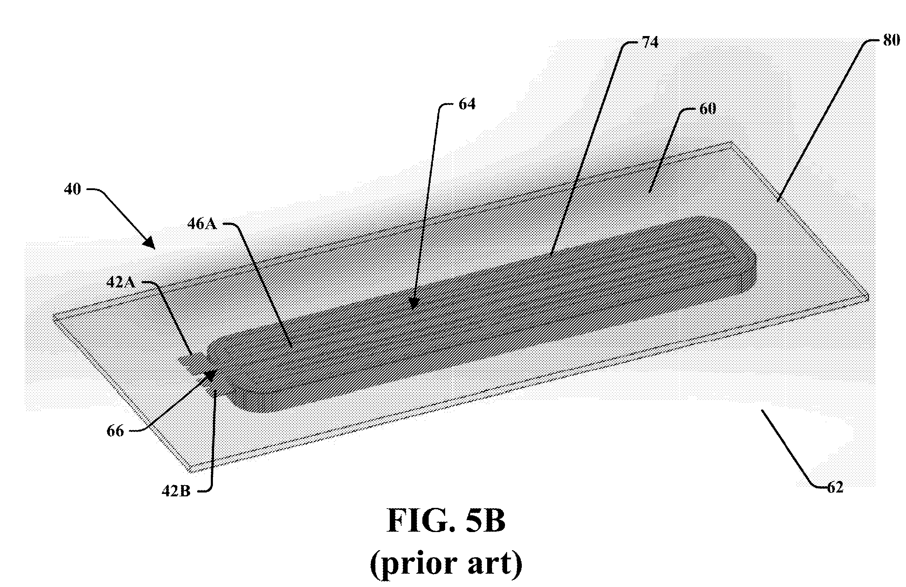

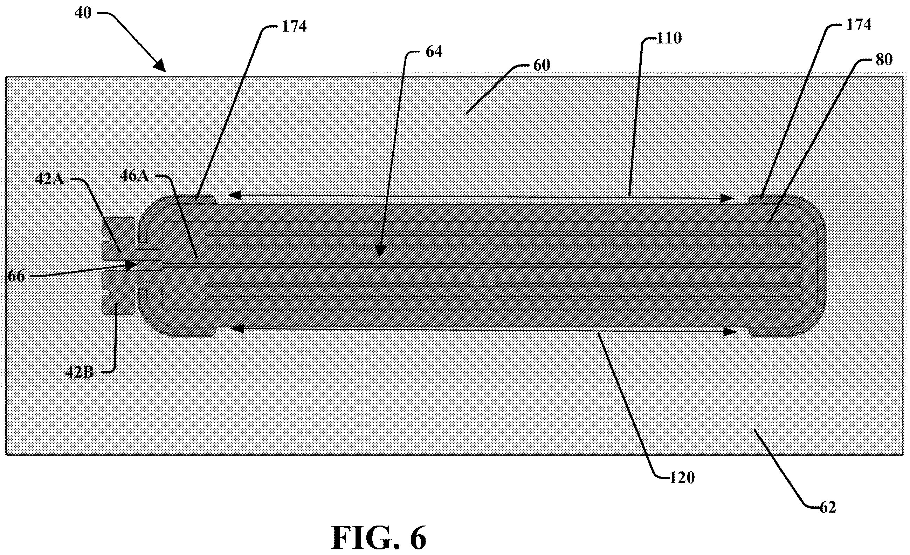

The capacitive gap 64 is vented to the outside of the sensor 40 by vent 66. The vent 66 may be a break in the bond or weld line between base 80 and diaphragm 60, which allows electrical traces or interconnect of the electrodes 46A to pass through and connect bond pads 42A, 42B. The vent is identified in Figures 5 and 6 adjacent the bond pads 42A, 42B and extending from the short side of the base 80 and diaphragm 60. The attachment configuration between the base 80 and the diaphragm 60 may define a discontinuity that allows at least one electrical trace to connect outside said capacitive gap 64 to at least one electrode positioned at least partially within the capacitive gap 64. The vent 66 may also allow the passage of fluid between the housing cavity 25 and the capacitive gap 64.

The underside 62 of the diaphragm 60 may be bonded to the base 80 along a continuous bond line 74 which is the attachment point about the perimeter of the capacitive gap 64. The sensor 40 may be a capacitive pressure sensor, wherein the diaphragm 60 may be designed to flex slightly and change the height of gap 64 when the diaphragm's top surface 68 and bottom surface 62 are exposed to different pressures. The diaphragm 60 and base 80 may be made from the same material or from different materials that are amenable to bonding and whose difference in thermal expansion coefficient may be such that the desired thermal properties may be obtained (either thermal stability or a known response to thermal changes). Materials for the diaphragm 60 and the base 80 may include glass, fused silica, quartz, sapphire, diamond, ceramic, silicon and its derivatives, germanium, SiGe and its derivatives.

Figure 5 is a top view of the sensor component 40 that illustrates how the bond line 74 surrounds the capacitive gap 64 except for the vent 66. Generally, the bond line 74 completely encircles the perimeter of the gap 64 (as a circle, square, oval, or some other shape). In this embodiment, the sensor 40 includes a parallel plate capacitor with a fixed electrode on the base 80 (which may be made of silicon) and a movable electrode on the diaphragm 60 (which may be made of glass) wherein external pressure on the implant 10 causes the glass diaphragm 60 to deflect, changing the capacitance and thus the implant's output frequency. Equation 1.1 below has been found to reflect this relationship:

$$f = \frac{1}{2\pi\sqrt{LC}} \text{ and } C = \frac{\varepsilon A}{d} \text{ - Equation 1.1}$$

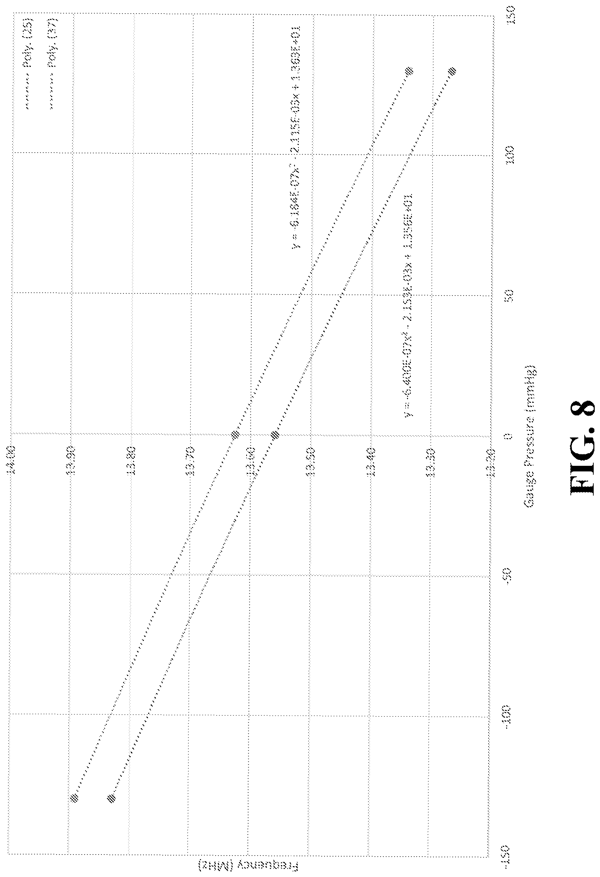

Here, $f$ is the implant's resonant frequency, L is the inductance of an attached inductor 30, $C$ is the capacitance and $d$ is the distance between the capacitor's electrodes 42A and 42B. In some embodiments, the implant 10 has experienced temperature induced offset of frequency between 80-90kHz. This type of frequency offset has been identified to correspond with smaller capacitance and with larger gaps between the electrodes. Figure 8 illustrates a graph that represents frequency v. pressure during simulations of the implant 10 of Figure 5. It illustrates that there exists a 66kHz frequency difference or error between the implant exposed to an environment of 25C temperature (upper curve) than when exposed to an environment of 37C temperature (lower curve). This translates to a 31.3 mmHg difference that may be due to gas expansion within the cavity 25 as well as different coefficients of thermal expansion between the glass diaphragm 60 and the silicon base 80.

Figure 6 illustrates an alternative embodiment of the implant 10. In this embodiment the sensor 40 is illustrated as a standalone unit as in previous embodiments but in this case the base 80 includes open portions or discontinuities 110, 120 along a discontinuous bond line 174 between the perimeter of the base 80 and the diaphragm 60. These open portions define spaces or discontinuities 110, 120 positioned along the discontinuous bond line 174 to provide a measured opening between the housing cavity 25 and the capacitive gap 64 defined between the base 80 and the diaphragm 60.

This configuration may be useful in either a parallel capacitor arrangement or a series capacitor arrangement. In parallel, one electrode may be positioned on the diaphragm 60 and one electrode on the base 80 and including the capacitive gap 64 therebetween and exposed to the cavity 25 of the housing 20 along discontinuities 110, 120 that extend along a measured portion of the length of the electrodes. The "series capacitor" embodiment includes two electrodes positioned along the diaphragm 60 and one electrode along the base 80. The parallel capacitor embodiment may provide twice the capacitance for the same electrode area and gap height as the serial capacitor embodiment which may provide an advantage in performance and design flexibility. The series capacitor may simplify fabrication of and simplify connection to the sensor 40. Both the series and parallel capacitor arrangements in addition to the discontinuous bond line 174 including discontinuities 110, 120 have been identified to have an effect of increased sensitivity, as well as frequency offset compensation.

The base 80, the diaphragm, or both may be formed to include the discontinuities 110, 120 thereon in an optimized configuration to establish the desired exposure of the capacitive gap 64 to the environment within the cavity 25 in accordance with the findings herein. The particular length and location of the discontinuities 110, 120 have been identified to provide a direct benefit of increasing sensitivity of the sensor 40. Additionally, the discontinuities 110, 120 provide indirect benefits, which allow for design tradeoffs to other features of the implant 10 that would otherwise decrease the sensitivity of the implant 10. For example, the discontinuities 110, 120 allow for the electrodes 46A, 46B to be smaller in order to increase Q value while maintaining a desired level of sensitivity. It is the smaller electrodes that improve Q value and the discontinuities 110, 120 that increase sensitivity. Likewise, the discontinuities 110, 120 may allow for increasing the height of the capacitive gap 64, or to increase diaphragm thickness in an effort to reduce a "pull-in" effect between the electrodes. Further, the discontinuities 110, 120 may be useful in preventing frequency offset that has been caused by exposing the implant 10 to various temperatures, such as between ambient temperature and the temperature within the body of a patient.

The discontinuities 110, 120 may be provided in the base 80 (fixed silicon electrode) to allow the diaphragm 60 (moveable glass electrode) to have a more compliant glass membrane by increasing pressure sensitivity (kHz/mmHg) and also allowing for the increase of the structural thickness of the diaphragm 60 while maintaining pressure sensitivity.

Further, it has been identified that a change in temperature may cause material deflection leading to change in capacitive gap due to (i) gas expansion inside the capacitive gap 64, or (ii) coefficient of thermal expansion (CTE) mismatch between the dissimilar materials (e.g. glass vs. silicon). As the implant is moved from room temperature (25C) to body temperature (37C), gas expansion within the implant may cause displacement of the sensor materials, leading to change of capacitive gap 64 height in a different direction than the displacement caused by CTE mismatch between the dissimilar materials. The experienced temperature difference causes bond line strain due to CTE mismatch which may cause the gap to decrease while gas expansion within the capacitive gap 64 may cause the gap height to increase. By introducing the discontinuities 110, 120 with an optimized length between the base 80 and the diaphragm 60, the net capacitance change due to these conflicting effects may be reduced or even canceled.

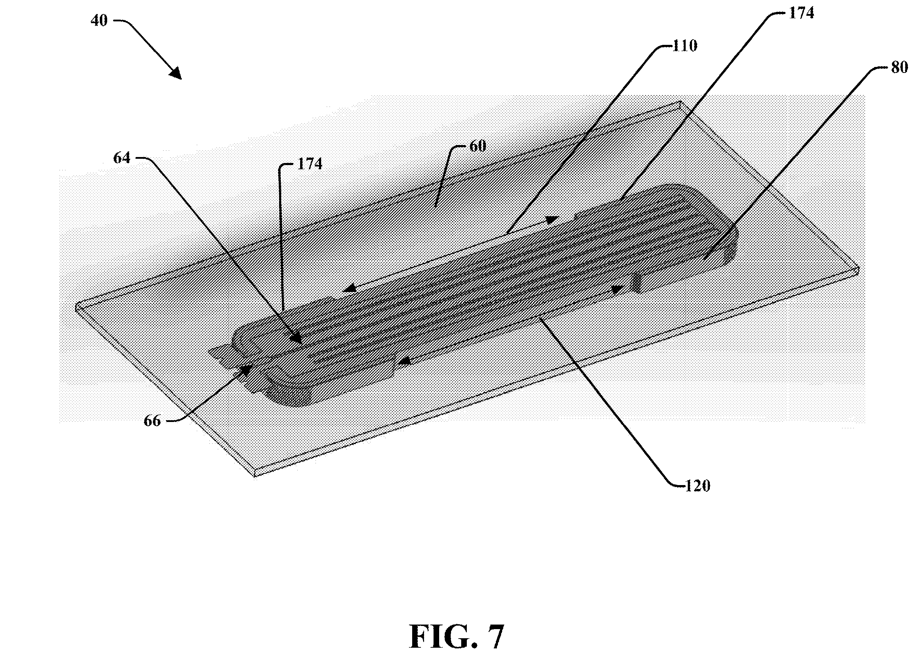

Notably, the embodiment of Figures 6 and 7 as well as the graphs illustrated by Figures 8-15 are directed to merely one embodiment of the implant and are provided by way of an example only as various different implant embodiments are contemplated by this application.

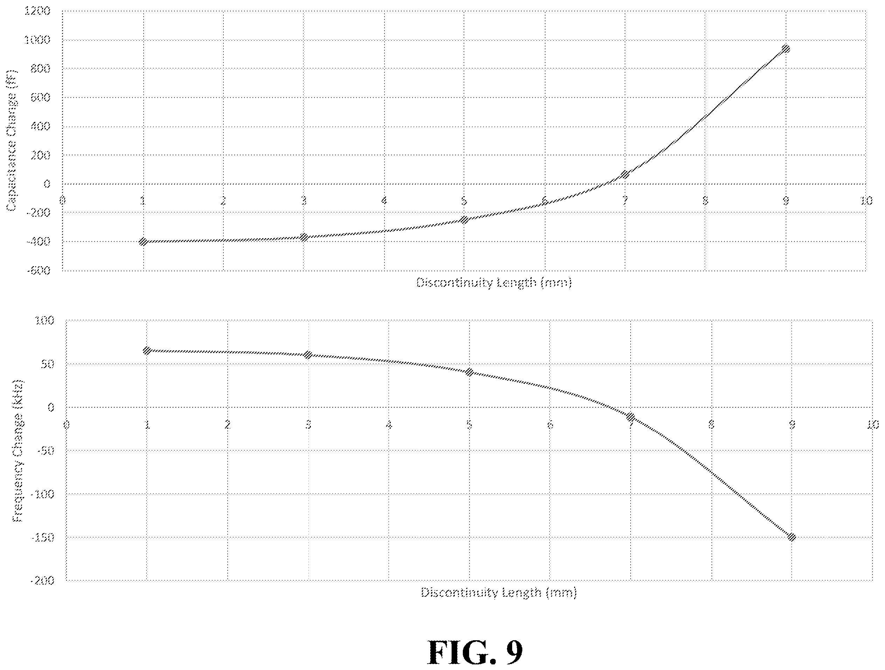

In one embodiment, an optimized length of the discontinuities 110, 120 may be determined by the graphs of Figure 9. Figure 9 includes a top graph that represents capacitance change vs. discontinuity length (of discontinuities 110, 120) of the implant 10 of Figure 6. The top graph illustrates that as the discontinuity length is 1mm, there is a capacitance change of about -400 fF. However, as the discontinuity length approached 6 or 7 mm, the change in capacitance approaches 0 fF. Further, as the discontinuity length is increased passed 7 mm, the capacitance change (fF) begins to increase to over 800 fF.

The bottom graph of Figure 9 represents the corresponding frequency change vs. discontinuity length of the implant 10 of Figure 6. The bottom graph illustrates that as the discontinuity length is 1mm, there is a frequency change over about 50 kHz. However, as the discontinuity length approached 6 or 7 mm, the frequency change approaches 0 kHz. As the discontinuity length is increased passed 7 mm, the frequency change begins to increase to over -150 kHz. Notably, the top and bottom graphs of Figure 9 illustrate various simulations performed over a range of temperature changes, observing the capacitance change and the frequency change relative to the discontinuity length.

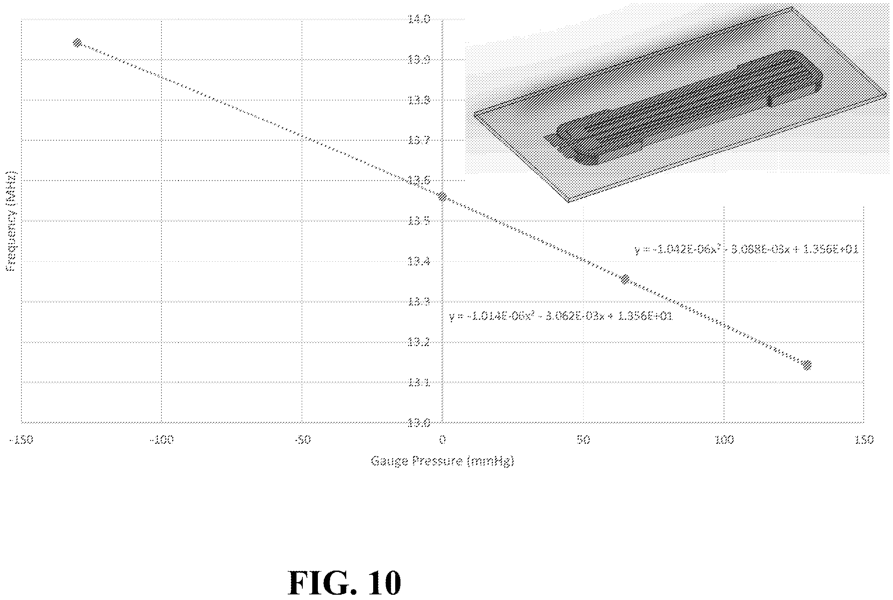

Figure 10 is a graph that represents frequency v. pressure during simulations of an embodiment of the implant 10 of Figure 6. Figure 10 illustrates an optimized embodiment that cancels the different displacements caused by the coefficient of thermal expansion of unrelated materials between the diaphragm 60 and the base 80. As in Figure 8, there are two curves on the graph, one representing implant performance at 25C and the other at 37C. However, the discontinuities 110, 120 in the Figure 10 graph of the sensor 40 have corrected the temperature offset seen in Figure 8, and now the two curves are nearly identical. In this embodiment, the diaphragm 60 is made from a glass material that includes a thickness of about 195 um and wherein the discontinuities 110, 120 are designed along the bond line 174 and include a length along the sides of the base 80 that are about 6.75mm. This optimized sensor 40 embodiment was found to have a capacitive change of about 9 fF and a frequency change of about -1.5 kHz when this sensor 40 was exposed to an environment of 25C and then to an environment of 37C. This configuration translates to a gauge pressure of 0.5 mmHg. This optimized sensor 40 accounts for gas expansion within the cavity 25 and thermal coefficient difference between the glass diaphragm 60 and the silicon base 80.

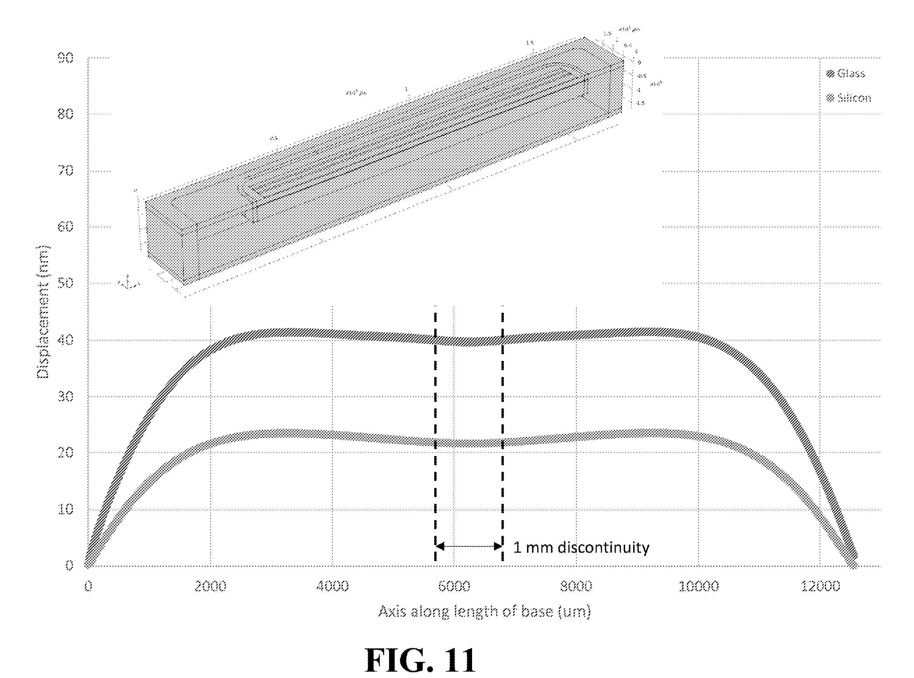

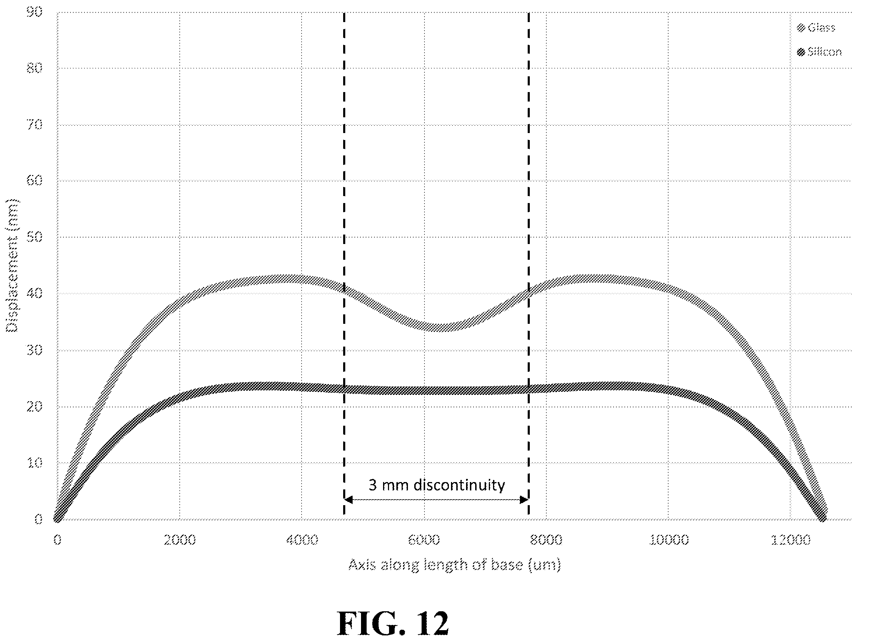

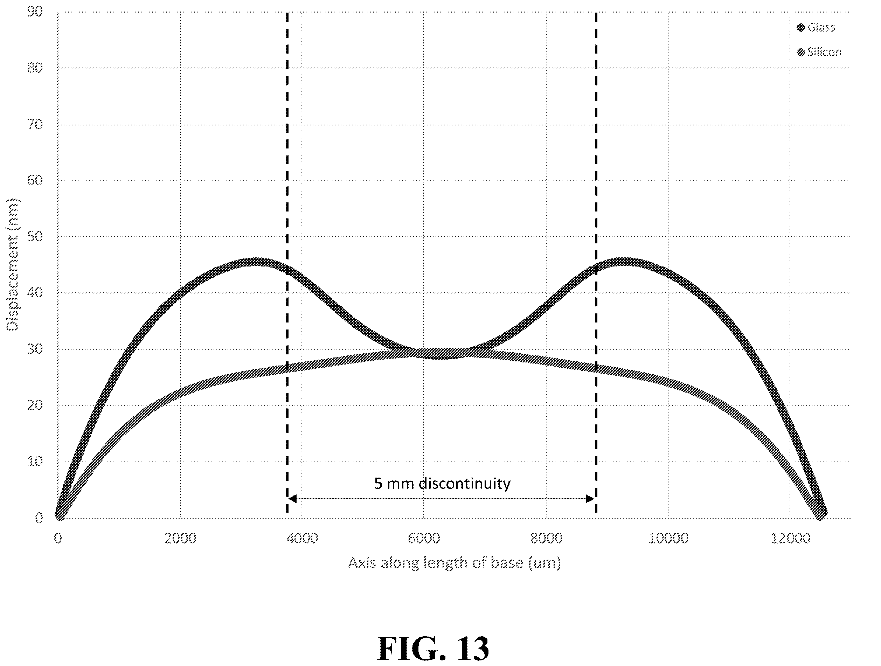

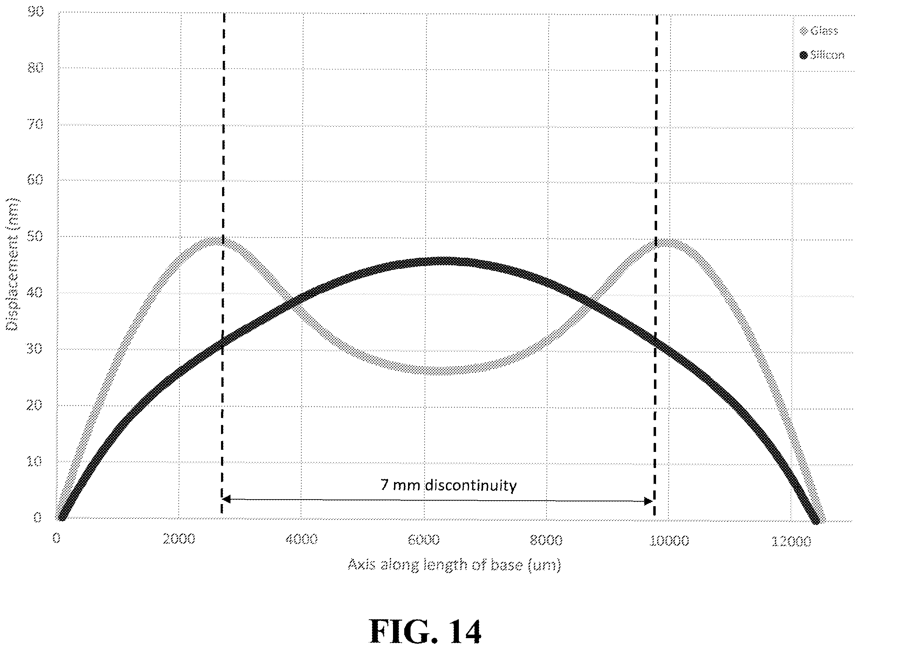

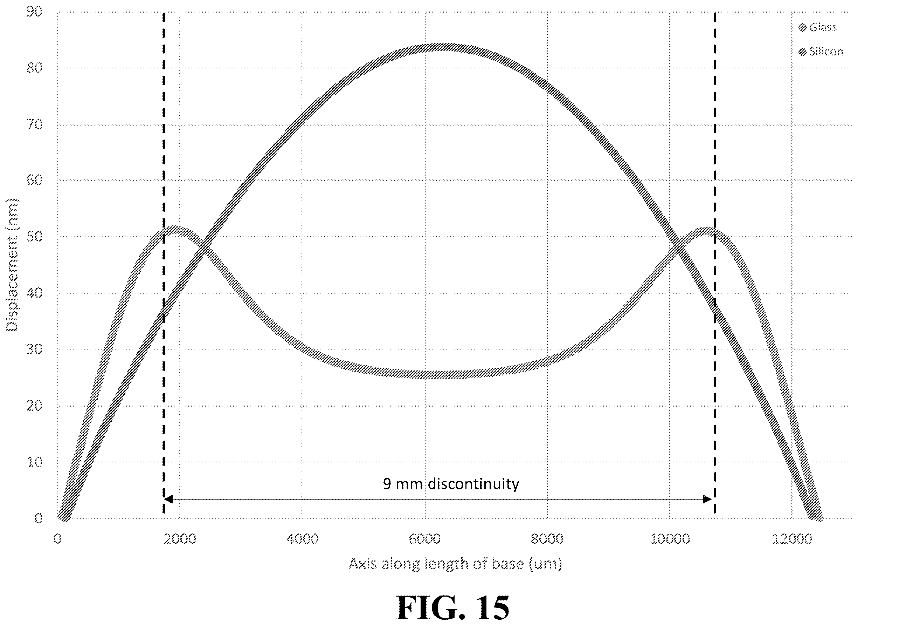

Figures 11 through 15 each include a graph that represents displacement vs. axis length during simulations of different embodiments of the implant 10 of Figure 6 wherein the discontinuities 110, 120 have a different length. Figures 11-15 illustrate the vertical displacement (nm) and location of displacement along the axis of the sensor 40 of the glass diaphragm 60 relative to the silicon base 80 of Figure 6. In each of Figures 11-15, the line labeled "glass" illustrates the difference in displacement (nm), at constant pressure, of the glass diaphragm 60 that occurs between 25C and 37C ambient temperature. The line labeled "silicon" illustrates displacement difference between 25C and 37C of the silicon base 80. Figure 11 includes a discontinuity length of 1 mm, Figure 12 includes a discontinuity length of 3 mm, Figure 13 includes a discontinuity length of 5 mm, Figure 14 includes a discontinuity length of 7 mm, and Figure 15 includes a discontinuity length of 9 mm. Considered together, Figures 11-15 illustrate the wide range of parametric variation that can be achieved by simply adjusting the lengths of the discontinuities 110, 120. Further, it was discovered that it may be possible to cancel out the displacement caused by both CTE difference experienced between dissimilar materials and the displacement caused by gas expansion as the implant is exposed to different temperatures (i.e. from room to body temperature). Here the glass diaphragm has a different coefficient of thermal expansion than the silicon base wherein Figure 14 illustrates an embodiment wherein the discontinuity length of about 7mm assists to reduce the material displacement experienced by the dissimilar materials due to their different CTEs. In Figure 14, the area under each curve is approximately equal, indicating the point at which the two temperature effects on capacitance are in balance. Each respective curve identifies measurements taken from the bottom surface of the glass diaphragm and a top surface of the silicon base.

All of the variations and embodiments discussed herein will accrue the benefits discussed herein from careful design of the bond line discontinuities 110, 120 of Figure 6. In addition to the benefits already discussed, it should be appreciated that any reduction in the length of the perimeter bond line 174 will also reduce the effects of stress caused by bonding, including sensor inaccuracy due to long term relaxation of stress, risk of cracking, delamination and other stress related effects.

The same benefits may accrue from other lengths, locations, and numbers of discontinuities besides those depicted in Figure 6. Discontinuities may occur on the short sides instead of, or in addition to the long sides of bond line 174, as well as at one or more corners of the perimeter. Discontinuities may be asymmetrical with respect to one another. For certain embodiments, a number of smaller discontinuities, for example a dotted-line pattern, may provide benefit. Other embodiments may benefit from retention of a length of bondline at the midpoint of the long side of the capacitive gap 64 of Figure 6, possibly intended to limit gap change near the center of the diaphragm, typically the region of largest deflection. Finally, there may be benefit in placing discontinuities that are partial; that is, their width is thinner than that of the retained portion of the perimeter bondline 174, so their stress profile is lower, but they do not vent the capacitive gap 64.

It is further observed that in a MEMS fabrication process, varying device performance parameters with a simple change, such as the discontinuity length, location, shape, and number, provides a benefit. Such a change may require a minimal number of mask changes, perhaps only a single mask, depending on the specific fabrication process used.

It is further observed that the retained portion of the bond line 174 may be made wider, to strengthen the bond between diaphragm 60 and base 80. This may offset any weakening of the bond caused by introduction of the discontinuities 110, 120.

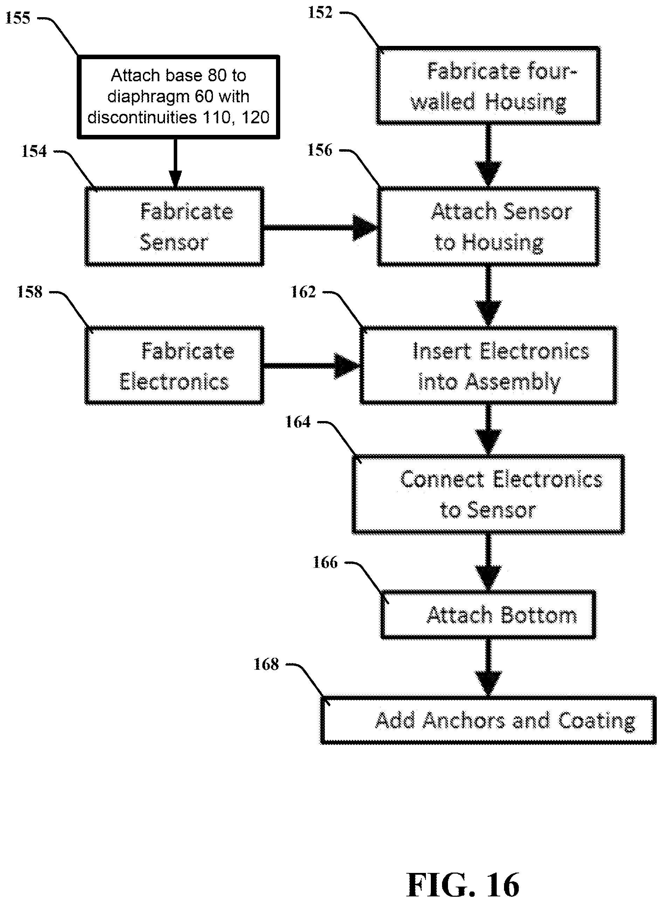

Figure 16 is a flowchart that illustrates an exemplary method for assembling the implant 10. Figure 16 represents one possible process for a typical embodiment of the implant 10. Some steps may not be necessary, or can be carried out in a different sequence, or may include other steps.

In step 152, an implant housing may be formed having four sides 52, 54, 56, and 58. However, this disclosure is not limited as other shaped housings 20 are contemplated, such as cylindrical, triangular, pentagonal, hexagonal, or any shape, including asymmetrical configurations. The sensor 40 may be fabricated as a standalone device, described by step 154. In one embodiment, a silicon wafer (base) is provided wherein a portion is etched away to partially form a cavity. An electrode may be applied to a surface of the silicon wafer within the partially formed cavity. The electrode may be formed by metal deposition and also include dielectric deposition. A glass wafer (diaphragm) may be provided that also includes an electrode thereon. The electrode may be formed by metal deposition. Here, in step 155, the silicon wafer base 80 may be formed to include discontinuities 110, 120 thereon in accordance with the findings herein. The shape and length of the implant 10 may assist in optimizing the length and location of the discontinuities 110, 120. The optimized length and location of the discontinuities 110, 120, such as determined by the graphs of Figure 9, have been identified to be useful in preventing frequency offset that has been caused by exposing an embodiment of the implant 10 to various temperatures, such as between ambient temperature and the temperature within the body of a patient. The glass wafer may be bonded to the silicon wafer along the bond line thereby forming the cavity and the capacitive gap with the aligned electrodes at least partially therein. Further etching, thinning, grinding, polishing, or dicing may occur to remove material from either the glass or silicon wafer to form the sensor 40.

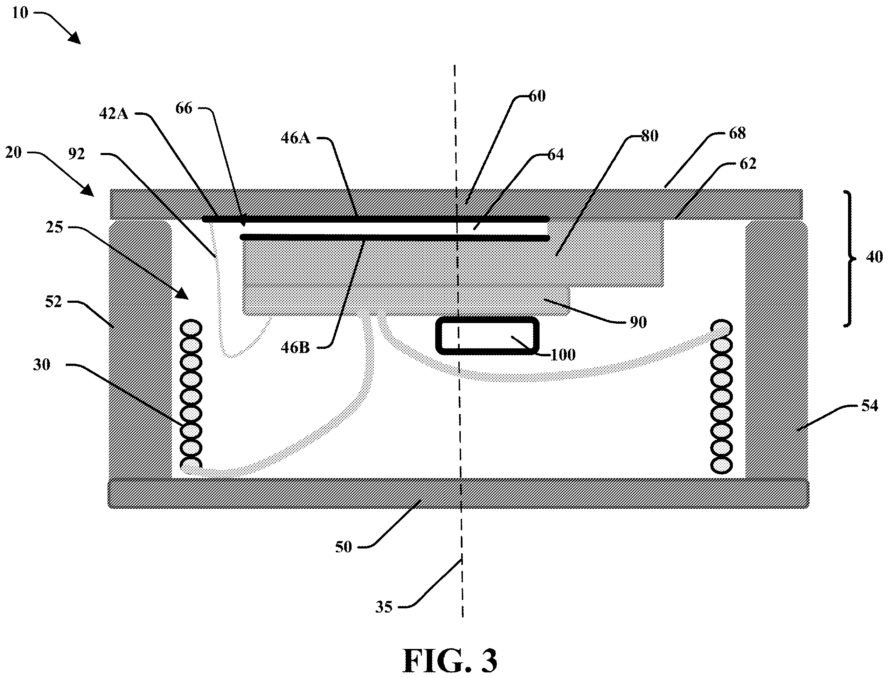

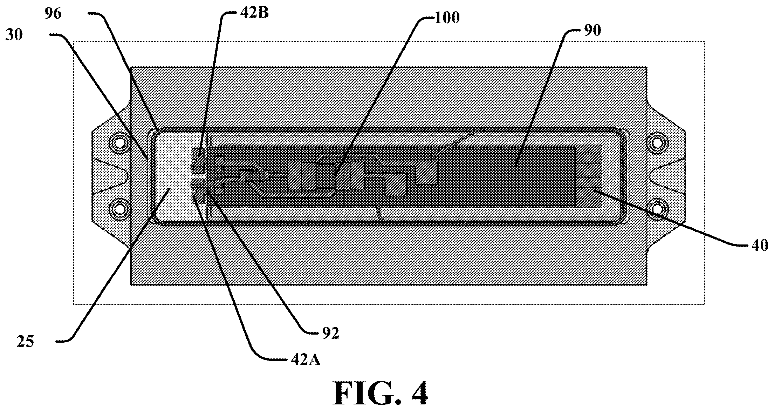

The sensor 40 may be attached to the housing 20 per step 156. Figures 3 and 4 illustrate the sensor 40 attached to the sidewalls 52 and 54 of the housing 20, such that the floating base 80 resides inside the cavity 25. The diaphragm 60 may have a perimeter that overlaps the base 80 sufficiently to attach to the top surfaces of the side walls by one of the hermetic attachment methods available.

The assembly or fabrication of electronics may occur in step 158. The implant 10 includes an antenna coil 30 that may be placed into the cavity 25 of the housing 20. Other electronic components, which may include one or more pressure sensors, may also be placed inside housing 20. The electronic components may be placed at least partially inside the region defined by the coil 30, or outside of this region. The electronic components may be positioned and attached along the inner surface 62 of the diaphragm 60. The coil 30 may be positioned such that it surrounds the floating base 80 and the electronic components partially or fully. The electronics and coil 30 may be assembled and interconnected electrically prior to insertion into the housing 20, or portions of the electronics and coil 30 may be inserted and then interconnected. By positioning the base 80 within the cavity 25 in a floating arrangement relative to the position of the coil 30, it may reduce the overall size of the implant 10. Additionally, during assembly, this configuration allows for accessibility to the electrodes without through vias or holes.

In the case where implant 10 contains a pressure sensor 40, the internal electronic components may include one or more pressure sensors such as MEMS pressure sensor components and the top wall 60 may be a diaphragm such as a flexible membrane. The top wall 60 and electrodes 46A, 46B may communicate pressure by slight vibrations. Also, a gas, a fluid, vacuum, desiccant material, getter material, or a gel may fill the cavity 25 formed by the housing 20. In another embodiment, the bottom wall 50 may also be a diaphragm such as a flexible membrane which may include additional electrical components that may also be part of a sensing electronic circuit (not shown). In either embodiment, pressure measurements may be transduced directly into an electronic signal of a sensing circuit or component.

The embodiments disclosed herein may benefit from having the final sidewalls attached in a vacuum environment or a known pressure. The cavity of the housing of the implant may be a sealed vacuum encapsulation. Additionally, the cavity of the housing may be sealed having a known pressure. This may assist to prevent internal pressures inside the housing from varying with temperature. Alternatively, the internal volume may be filled with an inert gas to limit corrosion of the internals. This may reduce the risk of problems related to moisture or other particulates.

In one embodiment, the implant sensor transmits a signal in response to an excitation pulse at a desired frequency range. In one embodiment, that frequency range is between 5 MHz to 30 MHz, and more particularly between 10 MHz to 20 MHz, or between 12 MHz to 15 MHz, and preferably between 13 MHz to 14 MHz.

## Claims

1. An implant (10) comprising:

   a housing (20) that defines a cavity (25);
   a sensor (40) connected to said housing (20) comprising:

      a diaphragm (60) having at least one diaphragm electrode (46A); and
      a base (80) attached to said diaphragm (60), said base (80) including at least one base electrode (46B) wherein said base (80) and diaphragm (60) define a capacitive gap (64) between the at least one diaphragm electrode (46A) and the at least one base electrode (46B), wherein the capacitive gap (64) is vented to the outside of the sensor (40) by a vent (66); and
      wherein said base (80) attaches to said diaphragm (60) along a perimeter of said capacitive gap (64), thereby forming an attachment, said attachment including, in addition to the vent (66), at least one discontinuity (110, 120) configured to enhance at least one performance parameter of said implant (10).

2. The implant (10) of claim 1, wherein said base (80) is positioned within the cavity (25) of said housing (20).

3. The implant (10) of claim 1, wherein said base (80) further includes at least one through hole to provide access to electrically connect said at least one electrode (46A, 46B) to a component outside said capacitive gap (64).

4. The implant (10) of claim 1, wherein said diaphragm (60) includes a thickness between about 100μm to about 300μm.

5. The implant (10) of claim 1, wherein said implant (10) includes a circuit having a resonant frequency that changes in response to a sensed parameter.

6. The implant (10) of claim 1, wherein said implant (10) is filled with a liquid or gel.

7. The implant (10) of claim 1, wherein said base (80) includes at least one through substrate via (TSV) to electrically connect the at least one of the base electrode (46B) and said diaphragm electrode (46A) to a component outside said capacitive gap (64).

8. The implant (10) of claim 1, wherein said diaphragm (60) includes a thick region and a thin region wherein said thin region is aligned with said capacitive gap (64).

9. The implant (10) of claim 1, wherein said diaphragm (60) is made of glass material and includes a thickness of about 195μm and said at least one discontinuity (110, 120) includes a length of about 6.75 mm and wherein said floating base (80) is made of silicon.

10. The implant (10) of claim 1, wherein said cavity (25) of the housing (20) of the implant (10) is filled with vacuum, inert gas, other gas species, or gas at a predetermined pressure.

11. The implant (10) of claim 10, wherein said diaphragm (60) is made of a glass material and said base (80) is made of silicon and wherein said performance parameter is sensitivity to temperature and is achieved by positioning said at least one discontinuity (110, 120) such that a coefficient of thermal expansion mismatch effect counteracts a gas expansion effect to reduce capacitive gap change due to temperature.

12. The implant (10) of claim 1, wherein said cavity (25) of the housing (20) of the implant (10) includes a getter material.

13. A method of assembling an implant (10) comprising:

providing a housing (20) that defines a cavity (25);

attaching a floating base (80) to a diaphragm (60) to form a capacitive gap (60), said attachment being positioned along a perimeter of said capacitive gap (64) and including at least one discontinuity (110, 120) to enhance at least one performance parameter of said implant (10), wherein the capacitive gap (64) is vented to the outside of the sensor (40) by a vent (66) different from the at least one discontinuity (110, 120);

attaching said diaphragm (60) to said housing (20) such that said floating base (80) is positioned within said cavity (25);

attaching a coil to said sensor (40); and

attaching a bottom to said housing (20) to form a hermetic seal about said cavity (25).

14. The method of claim 13, wherein said diaphragm (60) is hermetically attached to said housing (20) by at least one laser weld about the perimeter of said cavity (25).

15. The method of claim 13, wherein said diaphragm (60) is made of a glass material and said floating base (80) is made of silicon.

**Patentansprüche**

1. Implantat (10), umfassend:

ein Gehäuse (20), das einen Hohlraum (25) definiert;

einen Sensor (40), der mit dem Gehäuse (20) verbunden ist und umfasst:

eine Membran (60), die mindestens eine Membranelektrode (46A) aufweist; und

eine Basis (80), die an der Membran (60) befestigt ist, wobei die Basis (80) mindestens eine Basiselektrode (46B) umfasst, wobei die Basis (80) und die Membran (60) einen kapazitiven Spalt (64) zwischen der mindestens einen Membranelektrode (46A) und der mindestens einen Basiselektrode (46B) definieren, wobei der kapazitive Spalt (64) zur Außenseite des Sensors (40) durch eine Entlüftungsöffnung (66) entlüftet wird; und

wobei die Basis (80) entlang eines Umfangs des kapazitiven Spalts (64) an der Membran (60) befestigt wird und dadurch eine Befestigung bildet, wobei die Befestigung zusätzlich zu der Entlüftungsöffnung (66) mindestens eine Diskontinuität (110, 120) umfasst, die so ausgestaltet ist, dass sie mindestens einen Leistungsparameter des Implantats (10) verbessert.

2. Implantat (10) nach Anspruch 1, wobei die Basis (80) innerhalb des Hohlraums (25) des Gehäuses (20) positioniert ist.

3. Implantat (10) nach Anspruch 1, wobei die Basis (80) ferner mindestens ein Durchgangsloch zum Bereitstellen von Zugang zum elektrischen Verbinden der mindestens einen Elektrode (46A, 46B) mit einer Komponente außerhalb des kapazitiven Spalts (64) umfasst.

4. Implantat (10) nach Anspruch 1, wobei die Membran (60) eine Dicke von zwischen etwa 100 $\mu$m und etwa 300 $\mu$m umfasst.

5. Implantat (10) nach Anspruch 1, wobei das Implantat (10) eine Schaltung umfasst, die eine Resonanzfrequenz aufweist, die sich als Reaktion auf einen erfassten Parameter ändert.

6. Implantat (10) nach Anspruch 1, wobei das Implantat (10) mit einer Flüssigkeit oder einem Gel gefüllt ist.

7. Implantat (10) nach Anspruch 1, wobei die Basis (80) mindestens eine Durchkontaktierung durch das Substrat (TSV) zum elektrischen Verbinden mindestens einer, der Basiselektrode (46B) und/oder der Membranelektrode (46A), mit einer Komponente außerhalb des kapazitiven Spalts (64) umfasst.

8. Implantat (10) nach Anspruch 1, wobei die Membran (60) eine dicke Region und eine dünne Region umfasst, wobei die dünne Region mit dem kapazitiven Spalt (64) ausgerichtet ist.

9. Implantat (10) nach Anspruch 1, wobei die Membran (60) aus einem Glasmaterial besteht und eine Dicke von etwa 195 $\mu$m umfasst und die mindestens eine Diskontinuität (110, 120) eine Länge von etwa 6,75 mm umfasst, und wobei die schwebende Basis (80) aus Silizium besteht.

10. Implantat (10) nach Anspruch 1, wobei der Hohlraum (25) des Gehäuses (20) des Implantats (10) mit einem Vakuum, Inertgas, anderen Gasspezies oder einem Gas mit einem vorbestimmten Druck gefüllt ist.

11. Implantat (10) nach Anspruch 10, wobei die Membran (60) aus einem Glasmaterial besteht und die Basis (80) aus Silizium besteht und wobei der Leistungsparameter Temperaturempfindlichkeit ist und durch Positionieren der mindestens einen Diskontinuität (110, 120) derart bewirkt wird, dass ein Effekt einer Nichtübereinstimmung der Wärmeausdehnungskoeffizienten einem Gasausdehnungseffekt entgegenwirkt, sodass eine Änderung des kapazitiven Spalts aufgrund von Temperatur vermindert wird.

**12.** Implantat (10) nach Anspruch 1, wobei der Hohlraum (25) des Gehäuses (20) des Implantats (10) ein Getter-Material umfasst.

**13.** Verfahren zum Zusammenbau eines Implantats (10), umfassend:

Bereitstellen eines Gehäuses (20), das einen Hohlraum (25) definiert;
Befestigen einer schwebenden Basis (80) an einer Membran (60), sodass ein kapazitiver Spalt (60) gebildet wird, wobei die Befestigung entlang eines Umfangs des kapazitiven Spalts (64) positioniert wird und mindestens eine Diskontinuität (110, 120) zum Verbessern mindestens eines Leistungsparameters des Implantats (10) umfasst, wobei der kapazitive Spalt (64) zur Außenseite des Sensors (40) durch eine Entlüftungsöffnung (66), die sich von der mindestens einen Diskontinuität (110, 120) unterscheidet, entlüftet wird;
Befestigen der Membran (60) an dem Gehäuse (20), derart dass die schwebende Basis (80) innerhalb des Hohlraums (25) positioniert wird;
Befestigen einer Spule an dem Sensor (40); und
Befestigen eines Bodens an dem Gehäuse (20), sodass eine hermetische Dichtung um den Hohlraum (25) gebildet wird.

**14.** Verfahren nach Anspruch 13, wobei die Membran (60) mittels mindestens einer Laserschweißverbindung um den Umfang des Hohlraums (25) hermetisch an dem Gehäuse (20) befestigt wird.

**15.** Verfahren nach Anspruch 13, wobei die Membran (60) aus einem Glasmaterial besteht und die schwebende Basis (80) aus Silizium besteht.

## Revendications

**1.** Implant (10) comprenant :

un boîtier (20) définissant une cavité (25) ;
un capteur (40) relié au boîtier (20), comprenant :

un diaphragme (60) comportant au moins une électrode de diaphragme (46A) ; et
une base (80) fixée audit diaphragme (60), ladite base (80) incluant au moins une électrode de base (46B), ladite base (80) et ledit diaphragme (60) définissant un espace capacitif (64) entre l'au moins une électrode de diaphragme (46A) et l'au moins une électrode de base (46B), l'espace capacitif (64) étant ventilé vers l'extérieur du capteur (40) au moyen d'un évent (66) ; et
dans lequel ladite base (80) est fixée audit diaphragme (60) le long d'un périmètre dudit espace capacitif (64), formant ainsi une fixation, ladite fixation incluant, en plus de l'évent (66), au moins une discontinuité (110, 120) configurée pour améliorer au moins un paramètre de performance dudit implant (10).

**2.** Implant (10) selon la revendication 1, dans lequel ladite base (80) est positionnée dans la cavité (25) dudit boîtier (20).

**3.** Implant (10) selon la revendication 1, dans lequel ladite base (80) inclut en outre au moins un trou traversant destiné à fournir un accès pour la connexion électrique de ladite au moins une électrode (46A, 46B) à un composant situé à l'extérieur dudit espace capacitif (64).

**4.** Implant (10) selon la revendication 1, dans lequel ledit diaphragme (60) inclut une épaisseur comprise entre environ 100 $\mu$m et environ 300 $\mu$m.

**5.** Implant (10) selon la revendication 1, dans lequel ledit implant (10) inclut un circuit présentant une fréquence de résonance variant en réponse à un paramètre détecté.

**6.** Implant (10) selon la revendication 1, dans lequel ledit implant (10) est rempli d'un liquide ou d'un gel.

**7.** Implant (10) selon la revendication 1, dans lequel ladite base (80) inclut au moins un orifice traversant de substrat (TSV) pour la connexion électrique de l'une au moins parmi l'électrode de base (46B) et ladite électrode de diaphragme (46A) à un composant situé à l'extérieur dudit espace capacitif (64).

**8.** Implant (10) selon la revendication 1, dans lequel ledit diaphragme (60) inclut une région épaisse et une région fine, ladite région fine étant alignée avec ledit espace capacitif (64).

**9.** Implant (10) selon la revendication 1, dans lequel ledit diaphragme (60) est constitué d'un matériau en verre et inclut une épaisseur d'environ 195 $\mu$m, et ladite au moins une discontinuité (110, 120) inclut une longueur d'environ 6,75 mm, et dans lequel ladite base flottante (80) est constituée de silicium.

**10.** Implant (10) selon la revendication 1, dans lequel ladite cavité (25) du boîtier (20) de l'implant (10) est remplie de vide, d'un gaz inerte ou d'autres types de gaz, ou de gaz à une pression prédéterminée.

11. Implant (10) selon la revendication 10, dans lequel ledit diaphragme (60) est constitué d'un matériau en verre et ladite base (80) est constituée de silicium, et dans lequel ledit paramètre de performance est une sensibilité à la température et est atteint par positionnement de ladite au moins une discontinuité (110, 120) de manière à ce qu'un effet d'écart des coefficients de dilatation thermique compense un effet de dilatation du gaz afin de réduire une variation d'espace capacitif due à la température.

12. Implant (10) selon la revendication 1, dans lequel ladite cavité (25) du boîtier (20) de l'implant (10) inclut un matériau getter.

13. Procédé d'assemblage d'un implant (10) comprenant :

la mise à disposition d'un boîtier (20) définissant une cavité (25) ;
la fixation d'une base flottante (80) à un diaphragme (60) de manière à former un espace capacitif (60), ladite fixation étant positionnée le long d'un périmètre dudit espace capacitif (64) et incluant au moins une discontinuité (110, 120) destinée à améliorer au moins un paramètre de performance dudit implant (10), l'espace capacitif (64) étant ventilé vers l'extérieur du capteur (40) au moyen d'un évent (66) distinct de l'au moins une discontinuité (110, 120) ;
la fixation dudit diaphragme (60) audit boîtier (20) de telle façon que ladite base flottante (80) est positionnée dans ladite cavité (25) ;
la fixation d'une bobine audit capteur (40) ; et
la fixation d'un fond audit boîtier (20) afin de former un joint hermétique autour de ladite cavité (25).

14. Procédé selon la revendication 13, dans lequel ledit diaphragme (60) est fixé hermétiquement audit boîtier (20) par au moins une soudure au laser autour du périmètre de ladite cavité (25).

15. Procédé selon la revendication 13, dans lequel ledit diaphragme (60) est constitué d'un matériau en verre et ladite base flottante (80) est constituée de silicium.

**FIG. 1**

**FIG. 2**

**FIG. 3**

**FIG. 4**

**FIG. 5A**
**(prior art)**

FIG. 5B
(prior art)

**FIG. 6**

EP 3 740 120 B1

FIG. 7

**FIG. 8**

FIG. 9

FIG. 10

FIG. 11

**FIG. 12**

**FIG. 13**

Displacement (nm) vs. Axis along length of base (um)

Glass
Silicon

5 mm discontinuity

**FIG. 14**

**FIG. 15**

EP 3 740 120 B1

155 — Attach base 80 to diaphragm 60 with discontinuities 110, 120

152 — Fabricate four-walled Housing

154 — Fabricate Sensor

156 — Attach Sensor to Housing

158 — Fabricate Electronics

162 — Insert Electronics into Assembly

164 — Connect Electronics to Sensor

166 — Attach Bottom

168 — Add Anchors and Coating

**FIG. 16**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62618848
- US 21371216
- US 77765415
- US 2014030661 W
- US 61786793 B
- US 83707517
- US 129725
- US 9867552 B
- US 2012044998 W
- US 61502982 B
- US 8154389 B
- US 8493187 B
- US 213712
- US 20160324443 A1
- US 2012197155 A1
- WO 15213712 A